# EUROPEAN PATENT APPLICATION

(11) **EP 0 655 255 A1**
(43) Date of publication of application: **31.05.1995**
(21) Application number: 94308729.6
(22) Date of filing: 25.11.1994
(51) Int. Cl.: A61M 1/36

(54) **Side inlet bubble trap apparatus and method**

(30) Priority: 29.11.1993 US 158930; 29.11.1993 US 158928
(71) Applicant: COBE LABORATORIES, INC., Lakewood CO 80215-4407 (US)
(72) Inventor: Brugger, James M., Boulder, Colorada 80303 (US)
(74) Representative: Barlow, Roy James

(57) **Abstract**

An apparatus for trapping bubbles in blood flowing in a circuit, such as an extracorporeal circuit, includes a housing (32) which defines a substantially vertical chamber (68). Blood is introduced into the chamber through a delivery port (66) in a direction transverse to a longitudinal axis of the chamber. A deflector (70) is positioned in the chamber adjacent to and spaced apart from the delivery port. The deflector deflects at least a portion of the blood into the chamber above the deflector. Thereafter, the blood is removed from the chamber out an exit port (50) of the chamber. The deflection and redirection of blood within the chamber provides an opportunity for bubbles in the blood to separate from the blood in the chamber and also helps prevent the stagnation of blood in the chamber which can lead to clotting and separation of blood.

## Description

This invention relates to bubble trapping apparatus and methods for the treatment of a liquid. More particularly this invention relates to new and improved bubble trap apparatus and methods that aid in the removal of bubbles from blood and in the prevention of the formation of bubbles in the blood during its extracorporeal treatment.

Extracorporeal blood treatment involves removing blood from a patient, treating the blood external to the patient and returning the treated blood to the patient. Occasionally, bubbles form in the blood during extracorporeal blood treatment as a result of leakage of air into the blood at the point blood is withdrawn from the patient for extracorporeal treatment and as a result of leakage of air at points of connection in the extracorporeal treatment system. Bubbles also form as a result of turbulence of the blood flowing in the extracorporeal treatment system and coalescence of gases in the blood during treatment, among other causes. Care must be taken to remove bubbles from the blood prior to returning the blood to the patient and, to the extent possible, prevent formation of bubbles in the blood during treatment. Blood returned to the patient which contains bubbles creates a risk of serious health consequences to the patient.

Sometimes bubbles in blood flowing through extracorporeal treatment systems are optically or sonically detectable and many such systems incorporate equipment capable of detecting these bubbles. When bubbles are detected, the flow of blood returning to the patient is usually halted to prevent return of the detected bubbles to the patient. However, it is preferable that such bubbles be collected and removed from the blood so that blood treatment can continue uninterrupted.

Most extracorporeal treatment systems incorporate chambers for removal of bubbles from blood undergoing treatment. These chambers, often referred to as bubble traps, provide an opportunity for bubbles in the blood to separate from the blood while the blood is in the chamber. Bubbles in the blood rise to the surface of the blood in the chamber. Bubbles in the blood may also separate from the blood as the blood is delivered to the chamber, when the blood is delivered dropwise or in a stream over the surface of the blood already present in the chamber. The gas from the bubbles which collects above the level of blood is mechanically removed from the chamber, or is allowed to remain in the chamber until extracorporeal treatment is complete.

It is possible, however, that smaller bubbles may be present in the blood which may not be collected in the bubble traps of some extracorporeal treatment systems. The patient may then be subject to a risk of injury when these smaller bubbles coalesce into a larger bubble upon aggregation of the bubbles within the patient or may be subject to other risks.

Conditions under which bubbles form in the blood during extracorporeal treatment may be exacerbated by higher blood flow rates. For example, blood entering a bubble trap apparatus at a high rate may froth and create bubbles in the blood present in the bubble trap apparatus. These bubbles could pass through the bubble trap apparatus and remain in the blood to be returned to the patient.

Poor flow patterns can also create problems for blood flowing through a bubble trap apparatus. For example, if blood flow in a bubble trap apparatus is excessively turbulent, the blood's clotting processes may be activated undesireably and blood clots may form in the blood. Also by way of example, incomplete mixing of blood can cause blood in portions of the bubble trap apparatus to stagnate. The stagnated blood is then susceptible to clotting. Clotting of blood in a bubble trap apparatus may result in occlusion of lines of the extracorporeal blood treatment system or injury to the patient.

When blood is introduced into a bubble trap apparatus below the upper surface of the blood already present in the apparatus, stagnation and clotting have a tendency to occur in the blood near the upper surface of the blood. Stagnation and clotting occur near the upper surface of blood because the newly introduced blood tends to flow downward and often does not mix with blood above the point of introduction and near the upper surface. Although these clots may be filtered out of the blood before the blood is returned to the patient, excessive clot formation can occlude filters, which can lead to decreased blood flow to the patient.

One way that clot formation has been minimized is by introducing additional heparin into the blood of the patient. However, excess heparin in a patient can lead to other health problems and therefore excessive use of heparin is not preferred.

It is an aim of the present invention to overcome at least some of these problems and drawbacks.

### Summary of the Invention

One aspect of the present invention relates to a bubble trap apparatus in which large and small bubbles are more expeditiously removed from blood while the blood is being treated extracorporeally. Another aspect of the present invention relates to the minimization of bubble formation during such treatment. Still another aspect is the maintenance of blood flow throughout the apparatus to prevent stagnant areas from forming and thereby help prevent the formation of blood clots.

In accordance with these and other aspects, the present invention relates to an apparatus for trapping bubbles in blood flowing in a circuit, for example an extracorporeal blood treatment circuit, which comprises a housing defining a substantially vertical, longitudinal chamber. Blood is introduced into the chamber through a delivery port in a direction transverse to a longitudinal axis of the chamber. A deflector is positioned within the chamber to deflect at least a portion of the blood introduced into the chamber into a portion of the chamber above the deflector. After deflection, the blood flows generally downward along the length of the chamber and out an exit port of the apparatus. The deflection within the chamber and the flow of blood through the chamber provide an opportunity for bubbles in the blood to separate from the blood while the blood is in the chamber and help prevent stagnation and clotting of blood in the chamber.

In accordance with other aspects, the present invention relates to a method for removing bubbles from blood flowing through an apparatus. Blood is introduced into a substantially vertical chamber of the apparatus in a direction substantially transverse to the vertical axis of the chamber. At least a portion of the blood is deflected into the chamber above the level at which the blood was introduced in the chamber, to provide an opportunity for bubbles in the blood to separate from the blood and help prevent stagnation and clotting of blood in the chamber. Thereafter, the blood is removed from the chamber. An upper surface of the blood is maintained in the chamber above the level at which the blood is introduced to the chamber. Bubbles separating from the blood are collected above the upper surface of the blood.

These and other features of the present invention can be better understood from the following detailed description of preferred embodiments of the present invention meant merely by way of example, taken in conjunction with the accompanying drawings that are briefly described below.

Fig. 1 is a perspective view of an extracorporeal blood treatment system to which a patient undergoing blood treatment is connected, utilizing a bubble trap apparatus incorporating the present invention.

Fig. 2 is a perspective view of the bubble trap apparatus shown in Fig. 1.

Fig. 3 is a section view of the bubble trap apparatus taken substantially along the section line 3-3 of Fig. 2.

Fig. 4 is a top view of the bubble trap apparatus shown in Fig. 3.

Fig. 5 is a cross-sectional view of the bubble trap apparatus taken substantially along the section line 5-5 in Fig. 3.

Fig. 6 is a cross-sectional view of the bubble trap apparatus taken substantially along the section line 6-6 in Fig. 3.

Fig. 7 is a perspective view of another embodiment of the bubble trap apparatus which is an alternative to that shown in Fig. 2.

Fig. 8 is a section view of the bubble trap apparatus taken along the line 8-8 in Fig. 7.

Fig. 9 is a perspective view of another embodiment of the bubble trap apparatus which is an alternative to those shown in Figs. 2 and 7.

Fig. 10 is a section view of the bubble trap apparatus taken along the line 10-10 in Fig. 9.

### DETAILED DESCRIPTION

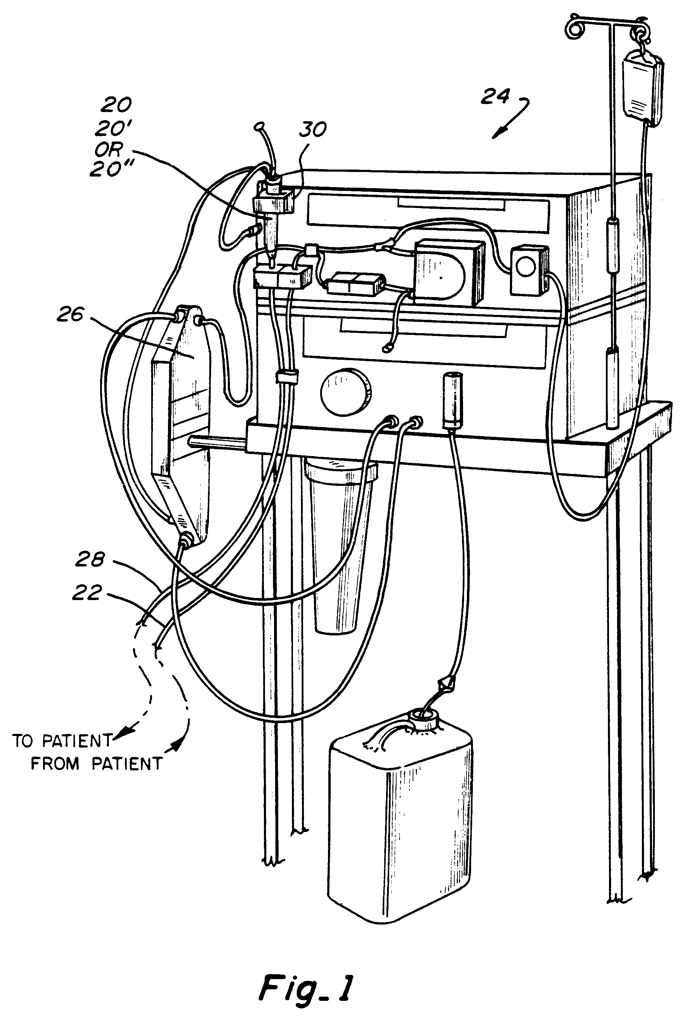

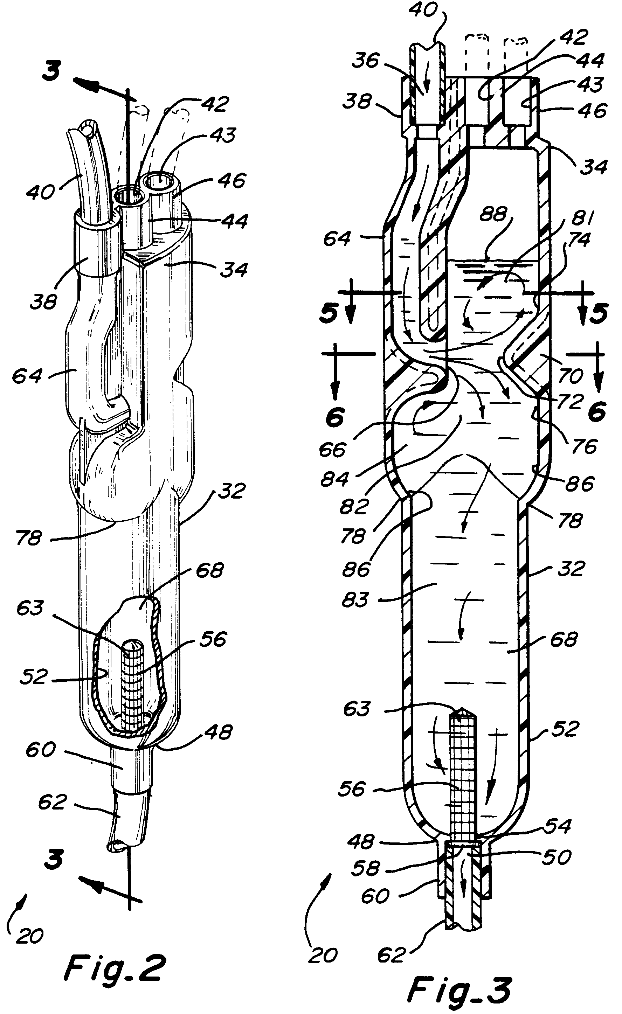

Three presently preferred embodiments of apparatus 20, 20' and 20'' for collecting bubbles in blood undergoing extracorporeal treatment are shown in Figs. 2 through 10. One bubble trap apparatus 20, 20' or 20'' is typically used as a component of an otherwise conventional extracorporeal treatment system 24, shown in Fig. 1.

Referring to Fig. 1, blood from a patient is circulated through conduit 22 to an extracorporeal treatment system 24 having a filtration unit 26 through which the blood flows. After passing through the filtration unit 26 the blood passes through the bubble trap apparatus 20, 20' or 20'' attached to the extracorporeal treatment system 24, after which the blood is returned to the patient via conduit 28. A blood level detector 30 of the extracorporeal treatment system 24 is operatively positioned relative to the apparatus 20, 20' or 20'' for detecting changes in the level of blood while the blood is in the apparatus 20, 20' or 20''.

Referring to Figs. 2 and 3, the bubble trap apparatus 20 includes a substantially elongated hollow housing 32. During use, the housing 32 is aligned vertically along a longitudinal reference or axis of the housing 32, and reference herein to the components of the housing 32 is in relation to the relative orientation of the components during such use as shown in Figs. 2 and 3. An inlet port 36 is formed in the upper end 34 of the housing 32. Blood is introduced to the apparatus 20 through the inlet port 36. As is shown in Figs. 2-4, an inlet coupling 38 is also formed in the upper end 34 of the housing 32 and surrounds the inlet port 36. The inlet coupling 38 receivably retains and is solvent bonded to a conduit 40 which is in fluid communication with the extracorporeal treatment system 24 (Fig. 1).

Also formed in the upper end 34 of the housing are first and second apertures 42 and 43. The first aperture 42 is surrounded by a first coupling 44 and the second aperture 43 is surrounded by a second coupling 46. Attached to the first coupling 44 is a device (not shown) for monitoring gas and pressure in the bubble trap apparatus 20. The second aperture 43 is typically used for introduction of medication or saline into blood flowing through the bubble trap apparatus 20.

An exit port 50 is formed in the lower end 48 of the housing 32. Blood exits the apparatus 20 through the exit port 50. A collar 54 is formed at the interior junction of the wall 52 of the housing 32 and the exit port 50. A filter 56 with a rim 58 having a diameter greater than the diameter of the collar 54 is inserted through the exit port 50 and is disposed within the apparatus 20, with the rim 58 of the filter 56 positioned below the collar 54. An exit coupling 60 surrounds the exit port 50 receives the rim 58 of the filter 56 and also receivably retains an exit conduit 62. The exit conduit 62 is in fluid communication with a return conduit (not shown). The exit conduit 62 is frictionally engaged by and solvent bonded to the exit coupling 60, with the filter 56 sandwiched between the exit conduit 62 and the collar 54, thereby preventing dislocation of the filter 56 and the exit conduit 62 during extracorporeal treatment. Perforations 63 are formed in the filter 56. The perforations 63 allow blood to pass through the filter 56 and out the exit conduit 62 but are small enough to block particulate matter later than a predetermined size, such as blood clots and foreign material, from passing into the exit conduit 62 and returning to the patient.

An inlet tube 64 is defined by upper portions of the wall 52 of the housing 32 and extends downward from the inlet port 36. The inlet tube 64 is hollow and extends approximately one third the length of the housing 32 to a point at which it turns perpendicularly towards the center of the housing 32 and becomes transverse to a vertically and longitudinally extending chamber 68 defined by the housing 32. Upon becoming transverse to the longitudinal chamber 68, the inlet tube 64 terminates at a delivery port 66 formed in the inlet tube 64.

The delivery port 66 of the inlet tube 64 opens into the chamber 68. The chamber 68 extends upward to the upper end 34 of the housing 32 and downward to the lower end 48 of the housing 32.

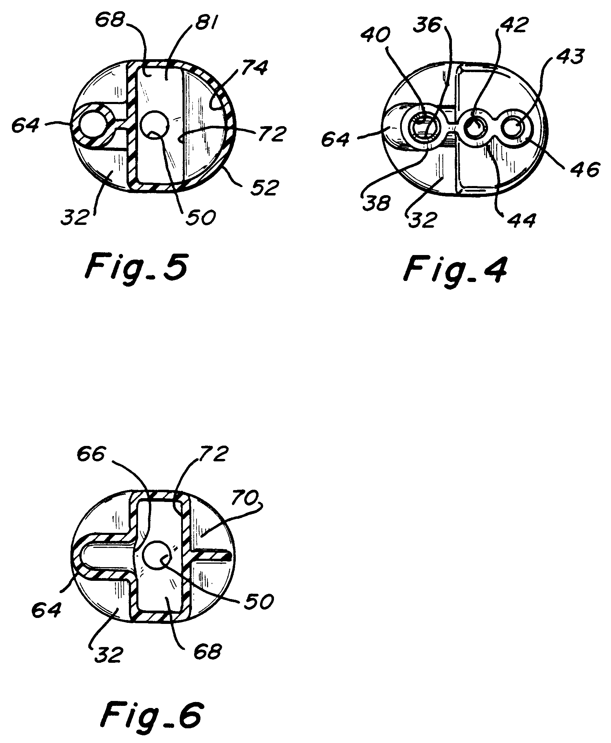

Formed in the wall 52 of the housing 32 and opposite the delivery port 66 of the inlet tube 64, is a deflector 70 (Figs. 3 and 6). The deflector 70 projects inwardly from the housing wall 52 into the chamber 68, effectively narrowing the chamber 68 at the center of the deflector 70 (Fig. 6). The deflector 70 is substantially wedge-shaped with an edge 72 projecting inward toward the chamber 68. The deflector 70 flares outward from the deflector edge 72 to merge into the upper inside surface 74 (Fig. 3, 4 and 5) and lower inside surface 76 (Fig. 3) of the housing wall 52, both of which inside surfaces 74 and 76 are substantially vertical.

The restricted size of the chamber 68 at the deflector edge 72 and a further narrowing of the chamber 68 below the deflector 70 at a neck 78 effectively divide the chamber 68 into first, second and third subchambers 81, 82 and 83 respectively (or a first subchamber 81 and a second subchamber which has first and second sub-subchambers 82,83). The first subchamber 81 is generally that portion of the chamber 68 above the deflector edge 72. Due to the flaring of the deflector 70 as it meets the upper inside surface 74 of the housing wall 52, the size of the first subchamber 81 is narrower near the deflector edge 72 and is wider towards the upper end 34 of the housing 32 (Fig. 5). The second subchamber 82 is generally that portion of the chamber 68 below the deflector edge 72 but above the neck 78 of the housing 32. Due to the flaring of the deflector 70 away from the deflector edge 72 as the deflector 70 merges into the lower inside surface 76 of the housing wall 52, and also due to the housing wall 52 extending downward and outward from the delivery port 66 of the inlet tube 64, the second subchamber 82 has a relatively wide midsection 84. The size of the second subchamber 82 narrows at the neck 78. The third subchamber 83 is defined at its upper extreme by the neck 78 and at its lower extreme by the lower end 48 of the housing 32. The third subchamber 83 is generally cylindrical with a substantially constant diameter, except near the lower end 48 of the housing 32 where the housing wall 52 curves gradually and smoothly inwardly to define the exit port 50.

As shown in Fig. 3, blood flowing in the conduit 40 during the extracorporeal treatment first flows through the inlet port 36 and thence downward along the inlet tube 64. The blood is then introduced into the chamber 68 at the delivery port 66, initially flowing in a substantially horizontal direction. Some of the blood entering the chamber 68 is deflected by the deflector 70 upward into the first subchamber 81 to mix with blood already present in the first subchamber 81. The gradual flare of the deflector 70 above the deflector edge 72 encourages blood to mix throughout the first subchamber 81 by directing blood to flow first along the upper flared surface of the deflector 70, then upward along the inside upper surface 74 of the housing wall 52 until the blood approaches the upper surface level 88 of the blood. Gravity then causes the blood to flow downward through the first subchamber 81, during which time the blood mixes with blood already deflected into the first subchamber 81. Stagnation and clotting of blood is substantially avoided in the first subchamber 81 by the absence of recesses and sharp corners in the first subchamber 81 in which blood might otherwise collect and by the fluid movement induced by the deflector 70.

Some of the blood entering the chamber 68 through the delivery port 66 is directed downward into the second subchamber 82 by gravity and by contact with and deflection off of the lower surface of the deflector 70 below the deflector edge 72. Other blood previously circulating in the first subchamber 81 flows downward into the second subchamber 82.

Blood in the second subchamber 82 is encouraged to mix by the narrowing size of the second subchamber 82 between the delivery port 66 and the deflector 70 and at the neck 78. The inwardly curving inner wall 86 of the housing 32 at the neck 78 directs blood flowing down along the inside housing wall towards the midsection 84 of the second subchamber 82. Some of the blood directed toward the midsection 84 is deflected by other portions of the curving inner wall 86, to mix with blood entering the second subchamber 82 from above. The absence of recesses and sharp corners in the second subchamber 82 substantially prevents stagnation of blood while the blood is undergoing mixing in and passing through the second subchamber 82.

Gases in the blood are given an opportunity to coalesce into bubbles, and these bubbles and bubbles already present in the blood are given an opportunity to float upward to the upper surface 88 of the blood in the first subchamber 81 while blood is in the chamber 68. In addition, bubbles present in the blood are urged upward toward the upper surface 88 of the blood in the chamber 68 by redirection of blood flow upward into the first subchamber 81 and circulation of blood upward within the first and second subchambers 81 and 82. The bubbles collect above the blood level 88 in the chamber 68. The upper surface 88 is preferably maintained in the first subchamber 81 at a level intermediate between the upper end 34 of the housing 32 and the deflector edge 72.

Gravity forces blood in the second subchamber 82 to flow downward into the third subchamber 83. After flowing the length of the third subchamber 83, the blood passes through perforations 63 in the filter 56, out the exit port 50 and into the exit conduit 62.

The neck 78 formed in the wall 52 of the housing 32 also serves to help align the bubble trap apparatus 20 during mounting to an extracorporeal treatment system 24 (Fig. 1). The wider portion of the apparatus 20 above the neck 78 contrasts with the narrower portion of the apparatus 20 below the neck 78 and functions as a stopper or wedge when the apparatus 20 is placed in a receiving clamp (not shown) of the extracorporeal treatment system 24. When functioning as a stopper, the widened portion of the apparatus 20 above the neck 78 prevents the apparatus 20 from incorrect alignment initially and from undesirably sliding downward during the course of the extracorporeal treatment.

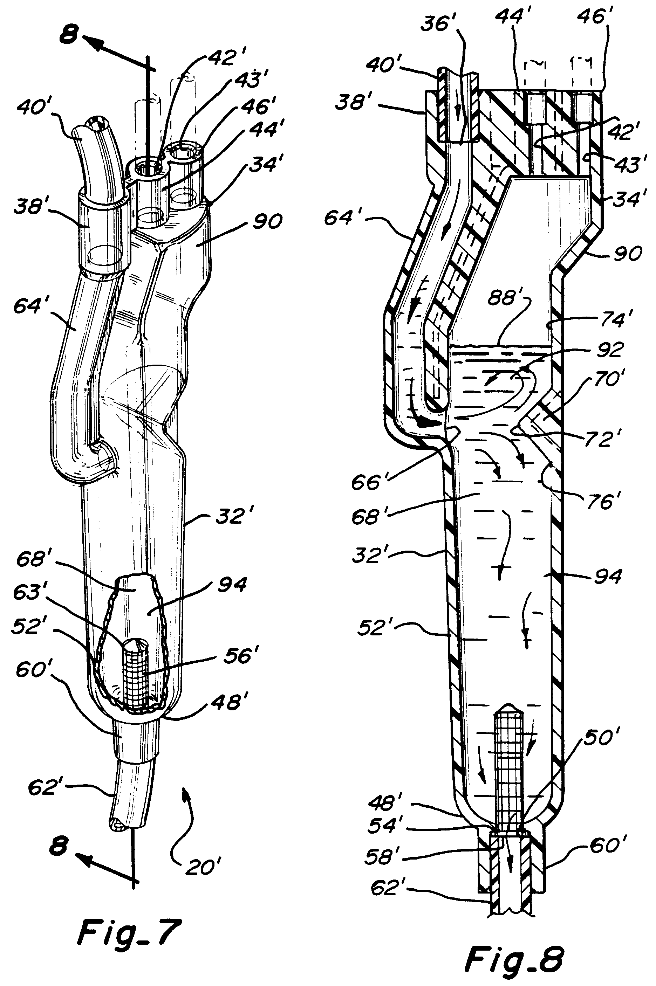

An alternate embodiment 20' of an improved bubble trap apparatus is shown in Figs. 7 and 8. The apparatus 20' includes similar features as those of the apparatus 20 which are referenced by like primed numerals. The apparatus 20' includes a substantially elongated hollow housing 32', albeit slightly bent at its upper end 34'. During use, the apparatus 20' is aligned vertically along a longitudinal reference or axis of the housing 32', and reference herein to the components of the housing 32' is in relation to the relative orientation of the components during such use. An inlet port 36' is formed in the upper end 34' of the housing 32'. Blood is introduced to the apparatus 20' through the inlet port 36'. An inlet coupling 38' is formed in the upper end 34' of the housing 32' and surrounds the inlet port 36'. The inlet coupling 38' receivably retains and is solvent bonded to a conduit 40' which is in fluid communication with an extracorporeal treatment system.

Also formed in the upper end 34' of the housing are first and second apertures 42' and 43'. The first aperture 42' is surrounded by a first coupling 44' and the second aperture 43' is surrounded by a second coupling 46'. A device (not shown) for monitoring gas and pressure in the bubble trap apparatus 20' is attached to the first coupling 44'. The second aperture 43' is typically used for introduction of medication or saline into blood flowing through the bubble trap apparatus 20'. An angled portion 90 of the housing 32' is adjacent to the second aperture 43' so that saline, medication or other material introduced into the chamber 68' through the second aperture 43' flows along the angled portion 90 rather than free falling into the blood in the apparatus and possibly causing undesirable spattering and frothing.

An exit port 50' is formed in the lower end 48' of the housing 32'. Blood exits the apparatus 20' through the exit port 50'. Formed at the interior junction of the wall 52' of the housing 32' and the exit port 50' is a collar 54'. A filter 56' with a rim 58' having a diameter wider than the diameter of the collar 54' is inserted through the exit port 50' and is disposed within the apparatus 20'. The rim 58' of the filter 56' is wider than the collar 54' and the filter 56' is fixedly positioned at the lower end 48' of the housing 32' adjacent to the exit port 50'. An exit coupling 60' surrounds the exit port 50' and receives the rim 58' of the filter 56' and also receivably retains exit conduit 62'. The exit conduit 62' is frictionally engaged by and solvent bonded to the exit coupling 60', with the filter 56' sandwiched between the exit conduit 62' and the collar 54', thereby preventing dislocation of the filter 56' and exit conduit 62' during extracorporeal treatment.

An inlet tube 64' is defined by upper portions of the wall 52' of the housing 32' and extends downward from the inlet port 36'. The inlet tube 64' is hollow and extends approximately one half the length of the housing 32' to a point at which the inlet tube 64' turns perpendicularly towards the center of the vertical longitudinal chamber 68' defined by the housing 32' and becomes transverse thereto. Upon becoming transverse to the longitudinal chamber 68', the inlet tube 64' terminates at a delivery port 66' formed in the inlet tube 64' and opens into the chamber 68'. The chamber 68' extends upward to the upper end 34' of the housing 32' and downward to the lower end 48' of the housing 32'.

A substantially wedge-shaped deflector 70' is formed in the wall 52' of the housing 32' and opposite the delivery port 66' of the inlet tube 64'. The deflector 70' projects inwardly from the housing wall 52' into the chamber 68' and terminates at a deflector edge 72', effectively narrowing the size of the chamber 68' at the edge 72' of the deflector 70'. The deflector 70' flares radially from the deflector edge 72' to merge into the upper inside surface 74' and lower inside surface 76' of the housing wall 52'.

The narrowed size of the chamber 68' at the deflector edge 72' effectively divides the chamber 68' into upper and lower subchambers 92 and 94. The upper subchamber 92 is generally that portion of the chamber 68' above the deflector edge 72'. Due to the flaring of the deflector 70' as it meets the upper inside surface 74' of the housing wall 52', the size of the upper subchamber 92 is narrower at the deflector tip 70' and wider where the deflector 70' meets the upper inside surface 74'. The lower subchamber 94 is generally that portion of the chamber 68' below the deflector edge 72'. The lower subchamber 94 is generally cylindrical with a substantially constant diameter, except at its upper end adjacent to the deflector 70' and near the lower end 48' of the housing 32' where the housing wall 52' curves gradually and smoothly inwardly to define the exit port 50'.

As is shown in Fig. 8, blood flowing through the apparatus 20' first flows through the inlet port 36' and thence downward along the inlet tube 64'. The blood is then introduced into the chamber 68' at the delivery port 66', initially flowing in a substantially horizontal direction. A portion of the blood entering the chamber 68' is deflected by the deflector 70' upward into the upper subchamber 92 to mix with blood already present in the upper subchamber 92. The gradual upward flare of the deflector 70' above the deflector edge 72' directs the blood upwards along the upper inner wall 74'. As the blood nears the upper surface 88' of the blood in the upper subchamber 92 the blood begins to flow downward, mixing with other blood present in the upper subchamber 92. Blood circulating in the upper subchamber 92 eventually flows downward into the lower subchamber 94. Stagnation and clotting of blood is substantially avoided in the upper subchamber 92 by the absence of recesses and sharp corners in the upper subchamber 92 in which blood might otherwise collect and by the fluid movement induced by the deflector 70'.

Some of the blood entering the chamber 68' is directed downward into the lower subchamber 94 by gravity and by contact with and deflection off of the lower surface of the deflector 70' which is below the deflector edge 72'. Gravity forces blood in the lower subchamber 94 to flow downward toward the exit port 50'. After flowing the length of the lower subchamber 94, the blood passes through perforations 63' in the filter 56', out the exit port 50' and into the exit conduit 62'.

Gases in the blood are given an opportunity to coalesce into bubbles, and the bubbles are given an opportunity to float upward to the blood level 88' in the upper subchamber 92 and while the blood flows through the upper and lower subchambers 92 and 94. Bubbles present in the blood flowing through the apparatus 20' are urged upward toward the surface of the blood in the chamber 68' by redirection of blood flow upward into the upper subchamber 92 and circulation of blood upward within the upper subchamber 92. The bubbles collect above the level of blood 88' in the upper subchamber 92, which is preferably maintained in the upper subchamber 92 at a level intermediate between the upper end 34' of the housing 32' and the deflector edge 72'.

The increased size of the upper and lower subchambers 92 and 94 of the chamber 68' as compared to the inside diameter of the inlet tube 64' means that blood flowing through the inlet tube 64' typically slows upon entering the chamber 68'. This decrease in blood flow rate makes it easier for bubbles in the blood to separate from the blood and float upward through chamber 68' to the upper blood surface 88' in the upper subchamber 92.

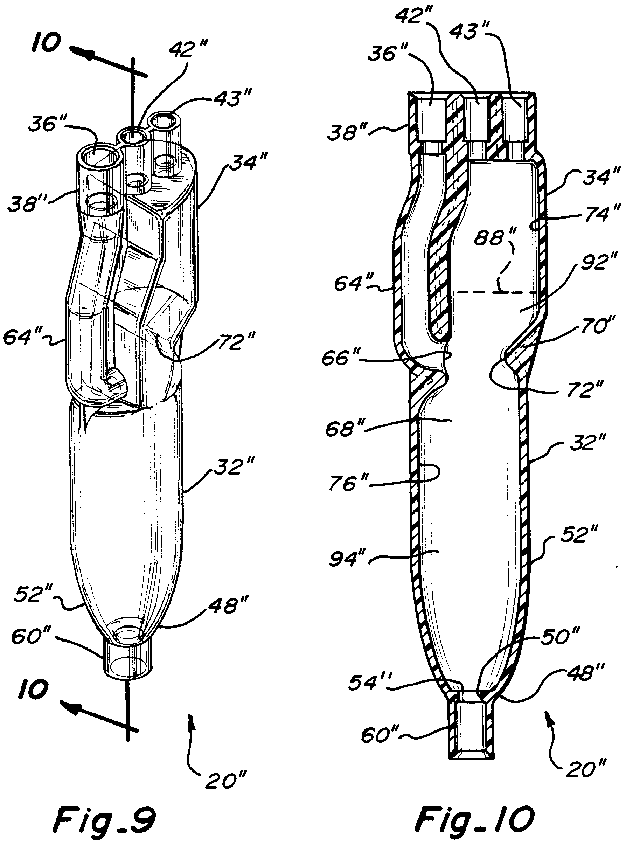

An alternate embodiment 20'' of an improved bubble trap apparatus is shown in Figs. 9 and 10. The apparatus 20'' includes similar features as those of the apparatus 20 and 20' which are referenced by like double-primed numerals. The apparatus 20'' includes a substantially elongated hollow housing 32''. During use, the apparatus 20'' is aligned vertically along a longitudinal reference or axis of the housing 32'', and reference herein to the components of the housing 32'' is in relation to the relative orientation of the components during such use. An inlet port 36'' is formed in the upper end 34'' of the housing 32'' through which blood is introduced to the apparatus 20''. An inlet coupling 38'', which is formed in the upper end 34'' of the housing 32'', surrounds the inlet port 36'' and receivably retains and is solvent bonded to a conduit (not shown) which is in fluid communication with an extracorporeal treatment system. Also formed in the upper end 34'' of the housing are first and second apertures 42'' and 43'', for purposes previously described in connection with apparatus 20 and 20'.

Referring to Fig. 10, an exit port 50'' is formed in the lower end 48'' of the housing 32'' through which blood exits the apparatus 20''. Formed at the interior junction of the wall 52'' of the housing 32" and the exit port 50'' is a collar 54''. Although not shown in Figs. 9 and 10, as described above in connection with the apparatus 20 and 20', a filter having a rim (not shown) is optionally positioned adjacent to the exit port 56'' to prevent blood clots and other materials from passing through the exit port 56'' in the blood being returned to the patient. An exit coupling 60'' surrounds the exit port 50''. Exit conduit (not shown) is frictionally engaged by and solvent bonded to the exit coupling 60'' to prevent dislocation of the exit conduit during extracorporeal treatment. If the optional filter is used, the rim of the filter is sandwiched between the exit conduit and the collar 54''.

An inlet tube 64" is defined by upper portions of the wall 52'' of the housing 32'' and extends downward from the inlet port 36''. The inlet tube 64'' is hollow and extends slightly less than one half the length of the housing 32'' to a point at which the inlet tube 64'' turns perpendicularly towards the center of the vertical longitudinal chamber 68'' defined by the housing 32'' and becomes transverse thereto. Upon becoming transverse to the longitudinal chamber 68'', the inlet tube 64'' terminates at a delivery port 66'' formed in the inlet tube 64'' and opens into the chamber 68''. The chamber 68'' extends upward to the upper end 34'' of the housing 32'' and downward to the lower end 48'' of the housing 32''.

A substantially wedge-shaped deflector 70'' is formed in the wall 52'' of the housing 32'' and opposite the delivery port 66'' of the inlet tube 64''. The deflector 70'' projects inwardly from the housing wall 52'' into the chamber 68'' and terminates at a deflector edge 72'', effectively narrowing the size of the chamber 68'' at the deflector edge 72''. The deflector 70'' flares upward from the deflector edge 72'' to merge into the upper inside surface 74'' and flares downward from the deflector edge 72'' to merge into the lower inside surface 76'' of the housing wall 52''.

The narrowed size of the chamber 68'' at the deflector edge 72'' effectively divides the chamber 68'' into upper and lower subchambers 92'' and 94''. The upper subchamber 92'' is generally that portion of the chamber 68'' above the deflector edge 72''. Due to the flaring of the deflector 70'' as it meets the upper inside surface 74' of the housing wall 52'', the size of the upper subchamber 92'' is narrower at the deflector tip 70'' and wider where the deflector 70'' meets the upper inside surface 74''. The lower subchamber 94'' is generally that portion of the chamber 68'' below the deflector edge 72''. The lower subchamber 94'' is generally cylindrical, except at its upper end adjacent to the deflector 70'' and towards the lower end 48'' of the housing 32'' where the housing wall 52'' tapers inwardly to define the exit port 50''.

As is shown in Fig. 10, blood flowing through the apparatus 20'' first flows through the inlet port 36'' and thence downward along the inlet tube 64''. The blood is then introduced into the chamber 68" at the delivery port 66'', initially flowing in a substantially horizontal direction. A portion of the blood entering the chamber 68'' is deflected by the deflector 70'' upward into the upper subchamber 92'' to mix with blood already present in the upper subchamber 92''. The gradual upward flare of the deflector 70'' above the deflector edge 72'' directs the blood upwards along the upper inner wall 74''. As the blood nears the upper surface 88'' of the blood in the upper subchamber 92'' the blood begins to flow downward, mixing with other blood present in the upper subchamber 92''. Blood circulating in the upper subchamber 92'' eventually flows downward into the lower subchamber 94''. Stagnation and clotting of blood is substantially avoided in the upper subchamber 92'' by the absence of recesses and sharp corners in the upper subchamber 92'' in which blood might otherwise collect and by the fluid movement induced by the deflector 70''.

Some of the blood entering the chamber 68'' is directed downward into the lower subchamber 94'' by gravity and by contact with and deflection off of the lower surface of the deflector 70'' which is below the deflector edge 72''. Gravity forces blood in the lower subchamber 94" to flow downward toward the exit port 50''. After flowing the length of the lower subchamber 94', the blood is directed towards the exit port 50'' by the tapered inner wall 52'' near the exit port 50'', and passes out the exit port 50'' and into the exit conduit.

Gases in the blood are given an opportunity to coalesce into bubbles, and the bubbles are given an opportunity to float upward to the upper surface 88'' of blood in the upper subchamber 92'' and while the blood flows through the upper and lower subchambers 92'' and 94''. Bubbles present in the blood flowing through the apparatus 20'' are urged upward toward the surface 88'' of the blood in the chamber 68'' by redirection of blood flow upward into the upper subchamber 92'' and circulation of blood upward within the upper subchamber 92''. The bubbles collect above the upper surface 88'' of the blood in the upper subchamber 92'', which is preferably maintained in the upper subchamber 92'' at a level intermediate between the upper end 34'' of the housing 32'' and the deflector edge 72''.

The increased size of the upper and lower subchambers 92'' and 94'' as compared to the inside diameter of the inlet tube 64'' means that blood flowing through the inlet tube 64'' typically slows upon entering the chamber 68''. This decrease in blood flow rate makes it easier for bubbles in the blood to separate from the blood and float upward through chamber 68'' to the upper surface 88'' of the blood in the upper subchamber 92''.

When either the apparatus 20' or 20'' is mounted in a level detector 30 of the extracorporeal treatment system 24 (Fig. 1), proper alignment of the apparatus 20' or 20'' within the level detector 30 is achieved, in part, by the narrowing of the housing 32' or 32'' at the point where the inlet tube 64'' or 64'' turns transversely toward the longitudinal axis of the housing 32' or 32'', respectively. In effect, the housing 32' or 32'' functions as a stopper, preventing downward movement of the apparatus 20' or 20'' during operation. By so aligning the apparatus 20' or 20'' within the level detector 30, a proper level of the upper surface 88' or 88'' is more likely to be maintained during extracorporeal treatment.

Each of the bubble trap apparatus 20, 20' and 20'' is preferably constructed of substantially transparent, flexible polyvinyl chloride using conventional blow molding techniques. Clear plastic is selected because it allows the operator to view blood level in the apparatus 20, 20' and 20'' during treatment. Flexibility of the polyvinyl chloride allows the apparatus 20, 20' or 20'' to be adjustably positioned in alignment with the level detector 30. Polyvinyl chloride is typically biocompatable and gamma-sterilizable and thus is suitable for bubble trap apparatus 20, 20' and 20'' through which blood flowing in an extracorporeal treatment system 24 passes.

Presently preferred embodiments of the present invention and many of its improvements have been described with a degree of particularity. It should be understood that the present invention is defined by the scope of the following claims.

## Claims

1. Apparatus for trapping bubbles in a liquid flowing in a circuit, said apparatus comprising:
a housing (32) having first and second housing ends (34,48) and defining an elongated chamber (68) extending along an axis between said two ends (34,48), preferably said axis being the longitudinal axis of said housing (32) or chamber (68);
delivery means (64) for introducing said liquid into the chamber (68); and
an exit port (50) formed in the housing (32) to allow said liquid to exit the chamber (68); characterised in that
said delivery means (64) introduces said liquid into the chamber (68) at a position intermediate said two housing ends (34,48); and further comprising
a deflector (70) positioned in the chamber (68) adjacent to and spaced apart from the position at which the delivery means (64) introduces said liquid into the chamber, the deflector (70) being arranged to deflect at least a portion of the said liquid introduced into the chamber (68) in the direction of the first housing end (34).

2. Apparatus according to claim 1, wherein said deflector (70) is arranged to deflect at least a portion of said liquid introduced into the chamber (68) in the direction of the second housing end (48).

3. Apparatus according to claim 1 or 2, further comprising:
a first subchamber (81,92) of the chamber (68), defined at its first extreme by the first housing end (34) and at its second extreme by the deflector (70), for mixing the deflected liquid and collecting bubbles which have separated from the liquid; and
a second subchamber (82,83;94) of the chamber (68), defined at its first extreme by the deflector (70) and at its second extreme by the second housing end (48), through which said liquid flows before removal through the exit port (50).

4. Apparatus according to claim 3 further comprising:
a collar (54) formed in the housing (32) and surrounding the exit port (50); and
a filter (56) having a rim (58), said filter (56) being positioned in the second subchamber (82,83) with the filter rim (58) abutting the collar (54), to filter said liquid in the second subchamber (82,83) before its removal through the exit port (50).

5. Apparatus according to any one of the preceding claims further comprising:
a neck (78) formed in the housing (32) at a point intermediate the deflector (70) and the second housing end (48), to cause liquid in said chamber (68) to mix in the chamber (81,82) on the second housing end (48) side of the deflector (70).

6. Apparatus according to any one of claims 1 to 4 wherein the deflector (70) has a deflector tip (72) and the apparatus further comprises:
a neck (78) formed in the housing at a point intermediate the deflector tip (72) and the second housing end (48).

7. Apparatus according to claim 5 or 6, when either claim is dependent on claim 3 or 4, wherein said second subchamber further comprises:
a first sub-subchamber (81) defined at its first extreme by the deflector (70) and at its second extreme by said housing neck (78), through which said liquid flows; and
a second sub-subchamber (82) defined at its first extreme by the housing neck (78) and at its second extreme by the second housing end (48), through which said liquid flows before removal through the exit port (50).

8. Apparatus according to any one of the preceding claims further comprising:
a first aperture (42) formed in the housing (32), preferably at the first housing end (34), for removing bubbles from the chamber (68).

9. Apparatus according to any one of the preceding claims further comprising:
a second aperture (43) formed in the housing (32), preferably at the first housing end (34), for adding fluid and materials to the liquid in the chamber (68).

10. Apparatus according to claim 9, further comprising:
an angled portion (90) of the housing (32) intermediate the first housing end (34) and the deflector (70), to help prevent splattering of the liquid in the chamber (68) when fluid or materials are added through the second aperture (43).

11. Apparatus according to claim 9 further comprising:
an angled portion (90) formed in the housing (32) near the first housing end (34) and the second aperture (43) to help prevent fluid and materials added to the liquid from splattering upon contact with the liquid in the chamber (68).

12. Apparatus according to any one of the preceding claims wherein said delivery means (64) is arranged to introduce said liquid into said chamber (68) in a flow direction substantially transverse to said axis of the chamber (68).

13. Apparatus according to any one of the preceding claims wherein the delivery means comprises:
an inlet tube (64) extending into the chamber (68) at a point intermediate the first and second housing ends (34,48) in a direction substantially transverse to said axis of the chamber (68).

14. Apparatus according to claim 13, wherein the inlet tube (64) and/or said deflector are/is formed integrally with said housing (32).

15. Apparatus according to any one of the preceding claims, wherein said deflector (70) is substantially wedge-shaped.

16. A method of removing bubbles from a liquid flowing through a bubble trap apparatus, said apparatus including a housing (32) having a chamber (68) extending substantially along a vertical axis between two ends of said chamber (68), preferably said axis being the longitudinal axis of said housing, said method comprising the steps of:
introducing said liquid into the chamber (68);
allowing bubbles in the liquid to separate from the liquid in the pool;
collecting the separated bubbles; and
removing the liquid from the chamber after the bubbles have been separated therefrom; characterised in that
said liquid is introduced into the chamber substantially transversely to said axis of the chamber (68), at a position intermediate the housing ends (34,48); and by the further step of
deflecting at least a portion of the liquid introduced into the chamber in a direction above the position where the liquid is introduced into the chamber (68), to form a pool of said liquid in the chamber (68).

17. A method according to claim 16 further comprising the step of:
maintaining an upper surface of the liquid within the chamber (68) above the position where the liquid is introduced into the chamber (68).

18. A method of removing bubbles from blood in an extracorporeal blood treatment circuit, comprising a method according to claim 16 or 17.
